**Europäisches Patentamt**

**European Patent Office**

**Office européen des brevets**

(19)

(11) Publication number : **0 318 571 B1**

(12)

# EUROPEAN PATENT SPECIFICATION

(45) Date of publication of patent specification : **17.03.93 Bulletin 93/11**

(51) Int. Cl.⁵ : **C12Q 1/56, C12Q 1/37,** **// G01N33/86**

(21) Application number : **88906168.5**

(22) Date of filing : **15.06.88**

(86) International application number : **PCT/SE88/00326**

(87) International publication number : **WO 88/10312 29.12.88 Gazette 88/28**

(54) DETERMINATION OF COMPONENTS ACTIVE IN PROTEOLYSIS.

(30) Priority : **18.06.87 SE 8702556**

(43) Date of publication of application : **07.06.89 Bulletin 89/23**

(45) Publication of the grant of the patent : **17.03.93 Bulletin 93/11**

(84) Designated Contracting States : **AT BE CH DE FR GB IT LI LU NL SE**

(56) References cited :
EP-A- 14 349
EP-A- 123 883
EP-A- 168 738
DE-A- 3 413 311
US-A- 3 539 450
US-A- 4 409 327
US-A- 4 543 335
US-A- 4 563 420

(73) Proprietor : **CHROMOGENIX AB** **Taljegardsgatan 3** **S-431 53 Mölndal (SE)**

(72) Inventor : **AURELL, Leif** **Bassängbacken 8** **S-430 40 Särö (SE)** Inventor : **FRIBERGER, Petter** **C. Wilhelmsonsvägen 7** **S-416 56 Göteborg (SE)**

(74) Representative : **Onn, Thorsten et al** **AB Stockholms Patentbyra Zacco & Bruhn** **P.O. Box 23101** **S-104 35 Stockholm (SE)**

## Description

This invention relates to a freeze-dried reagent combination, preferably for use in clinical diagnosis, which enables a simple and rapid determination of components active in proteolysis, for example proteases and especially proenzymes, inhibitors, cofactors and activators for proteases. The invention also concerns a new process for the preparation of such a reagent combination and use of this in the fields of coagulation and fibrinolysis.

Protein-cleaving enzymes, so-called proteases, have a plurality of important functions in the body. As examples of proteases the following can be mentioned: Trypsin and chymotrypsin which are secreted from the pancreas and participate in the digestion process; elastase, kallikrein and catepsin of various types and the enzymes of the complementary system which i.a. participate in reactions caused by inflammatory and allergic conditions; thrombin and the factors $VII_a$, $X_a$, $XI_a$ and $XII_a$ consisting of proteases which participate in the chain of reactions leading to formation of blood coagulum, and plasmin and exogenic urokinase which are proteases providing the dissolution of the blood cagulum.

Thus, diagnostic methods for measurement in vitro of protease activity are of a great importance in a plurality of clinical applications. Not only the amount of active protease but first of all the amount of proenzyme that can be converted to an active protease, and also the amounts of inhibitors and activators participating in proteolytic processes are measured. Of course such measurements are of great importance in investigations of pathological conditions. In certain treatments such as in surgery and/or medication, in many cases a determination of the quantities of certain of these substances is also first carried out.

Conventional methods for determination of components active in proteolysis, such as proteases and factors related thereto, for example proenzymes, inhibitors and activators, are based on so-called bioassays, immunologic reactions in vitro or utilization of biological proteins which proteins need not per se be natural substrates for the relative protease.

The disadvantages of the above methods are that they are time-consuming and laborious both in respect of performance and calibration, have a limited sensitivity and/or exhibit a lacking specificity and are also difficult to standardize.

In the last few years synthetic low molecular substrates have been developed based on amino acids or short peptides and provided with a usually photometrically, easily measurable marker which is easily split off by proteases. These substrates have to a large extent facilitated the methods of quantifying proteases as several of the disadvantages mentioned above have been eliminated by means of such substrates. (Hemker H.C. - Handbook of synthetic substrates, 1983, Martinus Nijhoff Publishers, Boston).

These substrates, especially such as are provided with a photometrically measurable marker, so-called chromogenic substrates, have thus enabled the development of methods having an improved specificity and reproducibility. Moreover, by these methods activity is measured instead of amount as, on the other hand, is the case with for example imnunologic methods and, thus, a linear ratio of studied parameter to measuring result is obtained as distinguished from log-log- and lin-log ratios mostly occurring in so-called bioassays.

Despite the advantages indicated above measuring methods based on splitting of such, usually chromogenic, substrates are still impaired by several shortcomings limiting the usefulness thereof.

As a rule, these so-called substrate methods comprise two-step reactions which include, on one hand, a merely biochemical reaction and, on the other hand, a reaction between a protease and a suitable substrate. Depending on which active component is to be determined the reaction processes can be illustrated as follows.

A. Determination of inhibitor

$$E_e + I \longrightarrow E_i - I + E_{e-i} \quad (E_e > E_i)$$

$$S-M \xrightarrow{E_{e-i}} S + M$$

B. Determination of cofactor

$$E_e + R \xrightarrow{C} E_c - R + E_{e-c}$$

$$S-M \xrightarrow{E_{e-c}} S + M$$

C. Determination of activator

$$P \xrightarrow{A} p + E_A \quad (P \text{ in excess})$$

$$S-M \xrightarrow{E_A} S + M$$

D. Determination of proenzyme

$$P \xrightarrow{A} p + E_p \quad (A \text{ in excess})$$

$$S-M \xrightarrow{E_p} S + M$$

Thus, the second reaction step means splitting of a substrate having a marker through the influence of a corrsponding enzyme, the amount of enzyme available for splitting consisting of the amount of active enzyme remaining after or being formed through the first reaction.

Thus, in all cases the amount of marker M released is proportional to the analytes I, C, A and P, respectively. The above abbreviations have the following meanings, concentrations being marked by index.

E = enzyme

I = inhibitor

S-M = substrate provided with marker

M = marker

S = substrate residue remaining after splitting off the marker

R = reactant reacting with the enzyme through the influence of a cofactor

C = cofactor

A = activator

P = proenzyme or enzyme compound which can be otherwise activated

p = residue from activation of a proenzyme/enzyme compound

As a rule, these two-step methods are time-consuming and laborious. As a plurality of pipetting steps are included and it is essential that the reaction times of the respective step are followed with a great skill, moreover, when carrying out the analyses. Furthermore, the two-step method will limit the number of tests that can be carried out manually in one and the same series.

Besides, the usefulness of the methods is impaired by comparatively short shelf life (one month or less) that, as a rule, reconstituted biochemical reagents have. Commercial reagents available at present for these analyses are usually packed so that there is enough for 20-200 analyses. Consequently the user should need, for economical reasons, carry out a corresponding number of analyses within the time of the reconstituted reagents being stable. So far, technique and costs have not permitted simple packages for separate tests as included reagents usually must be packed individually in one way or other, as in separate packages or separate rooms in a packing container ("compartmentalized").

At present there is only one commercially available package form where the reagents for a single test are physically separated in one and the same plastic container (ACA®, DuPont, USA). The container having a technically complicated design is merely useful in an instrument specially made for this purpose, that breaks the partitions between the reagent compartments automatically and adds samples and further reagents after preset times.

It is also possible to simplify handling of conventionally packed reagents to a certain extent by adapting reaction conditions and reactants so that the two-step process is converted to a one-step process. The two reactions are then allowed to proceed in parallel and in such a way that the analyte is still directly proportional to the photometer reading. This process is either carried out in such a way that several reagents are added immediately after one another - which does not reduce the number of pipetting steps - or that certain reagents are mixed (e.g. substrate and biochemical reagent) in advance. Pipetting is then facilitated but a still more unstable reagent (shelf life usually only one day) is obtained. Particularly the last-mentioned process has been used in automatic methods (see below).

One of the advantages of the substrate methods is their applicability to automatic analyzers for clinical use facilitating a change into a one-step method in the measuring process (Bergström K. and Lahnborg, G. Tromb. Res 1975, Vol. 6, 223-233; Kapke G.F. et al, Clin. Chem. 1982, Vol. 28, 1521-1524). In this way many of the disadvantages of the substrate methods are eliminated. However, an investment in automatic analyzers requires long test series and concentration on central units to become profitable, which means, however, that the period from sampling until the analysis results are obtained may become unacceptably long.

A comparatively complicated process of achieving one-step methods is described in EP-A2-0 168 738, pipetting steps and exact timing demands being avoided. However, the process described therein is substantially limited to the technically complicated method of applying to a fixed carrier matrix the necessary biochemical reagents and substrates either in separate processes using different solvents preventing reaction or by compartmentalizing. Moreover, an apparatus specially constructed for the purpose is required for reading the resulting color.

US 4 409 327 discloses improved determination of <u>low</u> heparin concentrations, the improvement being achieved by addition of urea and certain urea derivatives to a conventional reagent mixture. Although it is claimed that a reagent of this citation comprising thrombin or Factor $X_a$ and a chromogenic substrate at a pH of 6 to 9, is quite stable in the dry state and after addition of water can be stored for about a week, this is not verified. To the contrary, such a reagent should have low stability since the said pH range of the solution is the optimal pH range for the said enzymes. In the illustrative examples of the patent the reagent mixture used for determination of heparin activity in plasma does not include the chromogenic substrate. Initially, said mixture is incubated with the test sample <u>whereafter</u> the substrate is added and the desired reaction starts immediately.

DE-A-34 13 311 is concerned with determination of thromboplastin time. Thromboplastin is not an enzyme but is often contaminated with proteases. Thus, according to this patent a chromogenic substrate having high specificity for thrombin but low specificity for other proteases is used to obtain a thromboplastin time reagent having improved stability. In example 1 a lyophilized reagent comprising a chromogenic substrate and thromboplastin is disclosed. However, enzymatic activity is not present in the reagent but provided by the test sample.

EP-A-0 123 883 is concerned with a reagent for determination of activated partial thromboplastin time, which comprises a sulfatide, a phospholipid and a soluble calcium salt in combination with a chromogenic substrate specific for thrombin, which reagent may be lyophilized. This reagent does not include any component having enzymatic reactivity toward the chromogenic substrate. Thus, the substrate is not degraded by any other reagent component before use thereof.

Thus, it is the object of this invention to provide a reagent combination by means of which determinations of proteolytically active components can be easily carried out in a single step using the substrate method, yet the disadvantages indicated above being eliminated.

This object is achieved according to the invention by means of a freeze-dried reagent combination as defined in claim 1.

Thus, the invention relates to a freeze-dried reagent combination enclosed in a container and intended for a direct or indirect determination of a component active in a proteolysis through cleaving of a substrate included in the reagent combination and capable of being cleaved by a protease to produce a detectable response, characterized in that the reagent combination comprises in a substantially unreacted form all the reagents required for the determination, said reagents comprising a chromogeric substrate and a component having enzymatic activity towards said substrate and optionally one or more additives known per se and is prepared by freeze-drying in a way known per se of a solution containing all the constituents included in the freeze-dried reagent combination each in a substantially unreacted form, said freeze-dried reagent combination having substantially its original activity at reconstitution as in a buffer solution.

The reagent combination is preferably enclosed in the container in an amount sufficient for carrying out one separate single-step substrate method determination of proteolytically active components, for example proenzyme, protease activators, cofactors or protease inhibitors or their activators.

Accordingly the present reagent combination contains a substrate that can be cleaved by a protease to produce a detectable response. Depending on which proteolytically active component is to be determined the reagent combination also contains other reagents, usually selected from the following components: Proenzyme, protease activator, protease inhibitor, an activator for the inhibitor and a cofactor for proteolysis. The reagent combination preferably also contains additives, for example stabilizing additives as is explained more in detail below.

Useful substrates are all substrates that can be split by proteases to produce a detectable response. Particularly suitable substrates are such as give a photometrically measurable response, so-called chromogenic substrates. The above-mentioned synthetic substrates are then preferably used. Such substrates are commercially available for example from KabiVitrum AB Diagnostika, Mölndal, Sweden, for example under the commercial designations S-2251 (H-D-Val-Leu-Lys--pNA·2HCl), S-2337 (Nα-benzoyl-Ile-Glu(γ-piperidide)-Gly-Arg-pNA·HCl), S-2366 (<Glu-Pro-Arg-pNA·HCl), S-2390 (H-D-Val-Phe-Lys-pNA·2HCl) and S-2732 (Nα-succinoyl-Ile-Glu-(γ-piperidide)-Gly-Arg-pNA·HCl). In addition to these substrates which are described in the following illustrative examples further examples of substrates are reported in Table I below which are useful according to the invention. However, the said substrates are only illustrative but not limitative of the present invention.

Table I

| Analyte | Enzyme | Designation | Structure |
| --- | --- | --- | --- |
| 1. antifactor $X_a$ | F Xa | S-2767 x) | Boc-D-Arg-Gly-Arg-pNA |
| | | S-2775 x) | Succinoyl-D-Arg-Gly-Arg-pNA |
| | | CBS 31.39 xx) | $CH_3SO_2$-D-Leu-Gly-Arg-pNA |
| 2. Antithrombin | thrombin | S-2266 x) | H-D-Val-Leu-Arg-pNA |
| 3. antiplasmin | plasmin | S-2403 x) | <Glu-Phe-Lys-pNA |
| | | Chromozym-L xx) | Tosyl-Gly-Pro-Lys-pNA |
| 4. heparin | FXa | see antifactor Xa! | |
| 5. Fibrin monomer | plasmin | S-2466 x) | H-D-Gln-Phe-Lys-pNA |
| | | PL-1 xx) | H-D-Nle-CHA-Lys-pNA |
| 6. t-PA | plasmin | S-2390 x) | H-D-Val-Phe-Lys-pNA |
| | | Chromozym-P'L xx) | Tosyl-Gly-Pro-Lys-pNA |
| 7. FX | FXa | S-2732 x) | Succinoyl-Ile-Glu-(γ-piperidide)-Gly-Arg-pNA |
| | | S-2765 x) | Benzyloxycarbonyl-D-Arg-Gly-Arg-pNA |
| | | Spectrozyme Xa xx) | Methyloxycarbonyl-D-CHG-Gly-Arg-pNA |
| 8. Plasminogen | plasmin | S-2406 x) | <Glu-Leu-Lys-pNA |
| | | Chromozym-PL xx) | Tosyl-Gly-Pro-Lys-pNA |
| 9. Protein C | Protein $C_a$ | S-2288 x) | H-D-Ile-Pro-Arg-pNA |
| | | S-2401 x) | <Glu-Thr-Arg-pNA |

x) Manufaturer KabiVitrum AB
xx) Manufacturer Pentapharm AG, Basel, Schweiz

Abbreviations:

BOC = t-butyloxycarbonyl
CHA = cyclohexylalanin
CHG = cyclohexylglycin
Tosyl = p-toluene sulfonyl

The invention is generally applicable to the determination of proteolytically active components according to the basic principles for the substrate determination method provided such freeze-drying conditions can be established for a solution of the reagents under which conditions these components do not loose their activity, for example react with each other, but recover essentially their original activity after reconstitution.

So far it has been possible to establish suitable conditions for all reagent combinations of interest, especially in the fields of coagulation and fibrinolysis except the combination of reagents required for determination of $\alpha_1$-antitrypsin and F VIII. However, it is possible that such conditions possibly will be established also for these combinations after further experimentation.

The invention is well suited for determination of the proteolytically active components mentioned above for the substrate determination method. Thus, suitable reagent combinations are, when the proteolytically active component consists of a) an inhibitor, b) a cofactor, c) an activator or d) a proenzyme, a chromogenic substrate which can be split by protease in combination with a) a protease, b) a protease and a reactant which can react with the protease under the influence of the cofactor to be determined, c) a proenzyme or another activable enzyme compound and, respectively, d) an activator for the proenzyme. Embodiments of the invention preferred at present appear from the illustrative examples.

The present invention also relates to a process for preparation of the freeze-dried reagent combination which process is defined in claim 5.

More specifically, the invention relates to a process for preparation of the freeze-dried reagent combination, which process is characterized in that a solution containing all the reagents required for the determination and possibly one or more additives known per se is produced in a solvent such as water under conditions that are controlled in such a way that the reagents remain substantially unreactive in the resulting solution and in a subsequent freeze-drying of the solution; and that the solution is freeze-dried in the container in a way known per se, the freeze-dried reagent combination being obtained wherein the reagents are included in a substantially unreacted form and wherein the reagents have substantially original activity after reconstitution of the reagent combination, e g in a buffer solution.

It is essential according to the invention that all the reagents included in the freeze-dried combination are dissolved in the same solution at least in the very freeze-drying process. The process will be especially simple to carry out if a stock solution is first prepared from all reagents concerned under conditions controlled according to the invention, said stock solution also containing possible additives being thereafter distributed in containers and freeze-dried in these containers. However, it is of course also possible to prepare several stock solutions containing one or some of the reagents concerned and possible additives which solutions are thereafter brought together and freeze-dried simultaneously as one single solution in the respective container. Water is a suitable solvent even if additives of other solvents may be used, for example acetic acid, methanol and dimethyl sulfoxide.

According to a suitable embodiment of the invention such an amount of the solution (or solutions) is introduced into each container as is intended for one single test. A cuvette or a well in a microliter plate or the like is then preferably used as container to which a sample can be added after which the container is directly transferred to standard equipment for clinical analyses, for example for photometry.

According to the invention it is essential that the conditions in the preparation of the solution of all the reagents or in the combination of several solutions of reagents are adjusted, for example by providing the solvent with suitable additives of stabilizing soluble components and/or that the solvent is adjusted to such a pH-range, preferably by means of buffer salts, so that a mixture of such components usually reactive with each other can be dissolved in the preparation of the freeze-dried reagent combination without undesired but per se expected reactions taking place in the preparation. How the conditions are to be adjusted is dependent on the reagents included in the reagent combination and will be explained more in detail below.

The establishment of such controlled conditions has enabled simultaneous freeze-drying according to the invention of all the reagents in one single solution. The mixture of reagents in the final form of the product is present in a freeze-dried state without any special compartmentalizing of the components included in the mixture which mixture has a good stability, an immediate dissolution of all the components included in the reagent mixture being achieved upon addition of a sample dissolved in a buffer for the freeze-dried reagent combination.

Thus, through the invention a product is provided which enables the utilization of all the advantages of the substrate determination methods and by which further advantages are achieved. For example, the need of several pipetting steps is eliminated as only pipetting for handling of the sample is required. Likewise the demands on accuracy as regards keeping correct incubation and reaction periods are eliminated. Moreover, lower demands are made on the technical skill of the operator and no specially constructed instrument for using the reagent combination is required for carrying out the analysis.

Another advantage of the products according to the invention is a considerably longer shelf life than for the corresponding reconstituted reagents and that they can be standardized by the manufacturer which provides a considerably simplified process for calculating the test result in comparison with previously known processes of the same type which usually require the establishment of a standard curve.

The present freeze-dried reagent combination is thus extremely well suited for routine use utilizing current instruments for carrying out single tests in small and/or large clinics when these have no automated analyzer in operation and/or no qualified personnel in service. Moreover, the product according to the invention provides the possibility of obtaining a quick response in acute situations as the determination can be carried out locally ("bedside") and as no preparative work such as preparation of reagent solutions, establishment of standard curve or running of controls is necessary.

Moreover, the use of the product according to the invention often means a considerable saving of resources and material in comparison with current products available on the market, such as "kits", which are not suited for determination of a few samples or in so far as they are designed for performance of a single analysis, said performance requiring special equipment.

As pointed out above the reagent combination of the invention contains a substrate capable of being cleaved by proteases and in addition other reagents depending on which proteolytically active component one wishes to determine. Thus, the preparation of this product must take place under conditions that are controlled with respect to which reagents are to be included in the reagent combination, i.e. depending on which active component is to be determined. The process is explained in the following more in detail with respect to embodiments thereof that are important at present, viz. determination of inhibitors, cofactors, activators and proenzymes.

## I. Process for the preparation of a product for determination of inhibitors

Determination of the amount of protease inhibitor in a sample is based on the fact that a known and well-defined amount of an enzyme inhibited by the formation of 1:1-complex with the inhibitor is added to the sample and that the excess of enzyme is determined by addition of a known and well-defined amount of a suitable substrate.

According to the invention it has surprisingly been found that it is possible under conditions suited for production on a large scale to prepare a solution containing the enzyme as well as the substrate under conditions controlled in such a way that neither the enzyme nor the substrate is destroyed in the solution or in the process of freeze-drying. It is then extremely surprising that the enzyme does not react with the substrate in the preparation of the reagent combination although the enzyme and the substrate will achieve almost complete activity after reconstitution of the freeze-dried combination, for example by addition of a suitable buffer.

In determination of protease inhibitors the process of the invention is based on the fact that such a pH-range has been found within which the enzyme activity in respect of the substrate is negligible, however no denaturation of the enzyme or any hydrolysis of the substrate taking place. Such a range is preferably pH 3-5 and most preferably pH is 4.2. The optimum value within the range varies somewhat depending on which enzyme and which substrate are to be utilized in the process.

Furthermore, it is advantageous that such a buffer is utilized for adjustment of optimal pH, the salts of which leave at freeze-drying or are present after freeze-drying in such low amounts that the sample can be prepared before the analysis in currently used buffers having a pH of 6.5-9.5 so that optimal reaction conditions, i.e. pH 6.5-9.5, can be obtained for the substrate-enzyme reaction as well as for the enzyme-inhibitor reaction. Suitable buffers for the process of preparing reagents are weak organic acids such as formic acid or acetic acid mixed with the respective salt. Also other acids having a pKa-value in the same range such as citric acid, ascorbic acid etc. can be used but as a rule lower concentrations are required with the result that a considerably less stable pH is attained.

To avoid degradation processes during freeze-drying and during a following storage of the reagent mixture and in order to promote a rapid dissolution of the reagent mixture when adding the sample and minimizing reagent adsorption at the reagent container additives are suitably used, such as inorganic salts, inactive proeins, e.g. albumin, sugar, e.g. mannitol and/or surfactants such as polyethylene glycol (PEG, Carbowax® 8000 available from Union Carbide, USA) and Triton® X-100 (Rohm & Haas, USA).

As shown in the following examples the excellent results obtained when using the reagent combination according to the invention are due to the fact that it is possible to find reaction conditions for the analysis, for example pH, ionic strength and buffer salts, which are appropriate for both the reactions, i.e. in the determination of inhibitor for the substrate-enzyme reaction as well as for the inhibitor-enzyme reaction.

The choice of substrate is also of a great importance when utilizing the present invention. Besides the fact that the substrate, as pointed out above, must be cleavable by protease to give a detectable response it is also essential that such a substrate is selected for a certain reaction as reacts effectively enough with the enzyme in order that reasonable measuring conditions, preferably reaction times shorter than 10 min, will be achieved but which is still not a substrate effective enough to prevent the enzyme molecule from reacting with the inhibitor. This condition can partly be expressed with the bonding ability of the substrate to the enzyme, $K_m$, which should preferably be $2-8\times10^{-4}$ mol/l.

Commercially available synthetic substrates are usually utilized, for example those indicated in table I and such as are described in the illustrative examples. Suitable substrate concentrations are $1-20\times10^{-4}$ mol/l. The concentration should be so high that there is a linear relationship between the enzyme concentration and the marker concentration. In general a preferred substrate concentration is $5\times10^{-4}$ mol/l.

However, it should be pointed out that an optimal substrate concentration and the affinity properties of the substrate are influenced by additives in the reaction mixture, and therefore strict general selection criteria cannot be defined.

Of course the choice of enzyme is dependent on which inhibitor is to be determined. Such enzymes are commercially available for a plurality of inhibitors of clinical interest, for example for several important protease inhibitors occurring in plasma such as antithrombin which can be determined via a reaction with thrombin or factor Xa in the presence of heparin, antiplasmin, which can be determined via plasmin, and kallikrein inhibitors, and according to the invention freeze-dried reagent combinations for carrying out these determinations can be prepared.

## II. Process for the preparation of a product for determination of cofactors

Cofactors comprise any different types of substances actuating enzyme reactions. Here a factor is exemplified in the first place which actuates the inhibition of certain serine proteases, primarily $FX_a$ and thrombin, with the inhibitor antithrombin, viz. heparin. The method for determination of heparin and similar substances is similar to the inhibitor method described above except that the inhibitor, in this case antithrombin, must be added in excess.

Due to this the enzyme amount rust be considerably increased in order that correct reaction conditions might be obtained. Otherwise the process is based on the same principles as the inhibitor method above exemplified for antithrombin and factor $X_a$. Thus, the same conditions apply as in the process for preparation of a reagent combination for inhibitor determination according to the process I.

Another cofactor that can be determined is fibrin monomer. This is a plasma protein converted by a coagulation enzyme and being a cofactor in the activation of the proenzyme plasminogen via the enzyme t-PA (plasminogen activator) which then serves as an activator. This enzyme i.e. the activator, is freeze-dried together with plasminogen, i.e. its substrate, and a chromogenic substrate suitable for the activation product, viz. the enzyme plasmin. The process for the preparation of the reagent combination corresponds to the process for preparation of a reagent combination for the inhibitor determination described above with the difference that the pH-range that can be used for the freeze-drying also comprises neutral pH and, thus, pH is suitably 3-8.5, and preferably 5-7.

## III. Process for the preparation of a reagent combination for determination of activators

The method for determination of activator is built on the principle of freeze-drying a suitable proenzyme together with a substrate suitable for the activated proenzyme in the presence of additives promoting stability and solubility in analogy with what has been described above. Moreover, certain determination methods can require the presence of reaction promoting reagents. Thus, it is possible according to the invention to freeze-dry simultaneously all the reagents that are required for determination of for example t-PA, viz. plasminogen, plasmin substrate and stimulator of fibrin or polylysin type, and still maintain their activity, the conditions previously described being used which in analogy with the process II above also comprise neutral pH. Thus, a suitable pH-range is 3-8.5 and preferably 6-7.

## IV. Process for the preparation of a reagent combination for determination of proenzymes

Proenzymes such as Protein C, plasminogen and factor X are well suited for determination by means of the substrate technique in the one-step embodiment provided the activator utilized as reagent in the presence of substrate for the corresponding activated proenzyme is fast-acting. Thus, the process according to this embodiment of the invention comprises freeze-drying of a substrate together with an activator that activates the major portion of proenzyme aoccurring i the sample within some minute. The resulting reagent combination is intended for determination of proenzyme, the sample being diluted so much that inhibitors of the activated proenzyme occurring by nature in the sample are present in such a low concentration that these do not disturb the reaction between activated proenzyme and its substrate.

According to the invention freeze-drying is carried out in conventional manner, e.g. at 0,08-0.15 mbar by freezing at a temperature of -45° to -40°C, preferably -42°C for a time of 1-5 h, preferably 1 h, and a following drying for a time of 12-20 h, preferably 15 h, at a temperature of 12-24°C, preferably 22°C.

The invention also relates to use of a freeze-dried reagent combination for quantitative, semi-quantitative or qualitative determination of coagulation factors and fibrinolysis factors in a sample, especially a biological one, such as whole blood, blood plasma, blood serum, cerebrospinal liquid, lung liquid or urine, in a one-step process by addition of the sample to the freeze-dried reagent combination enclosed in a container, preferably a cuvette, and reading in a way known per se of a response received.

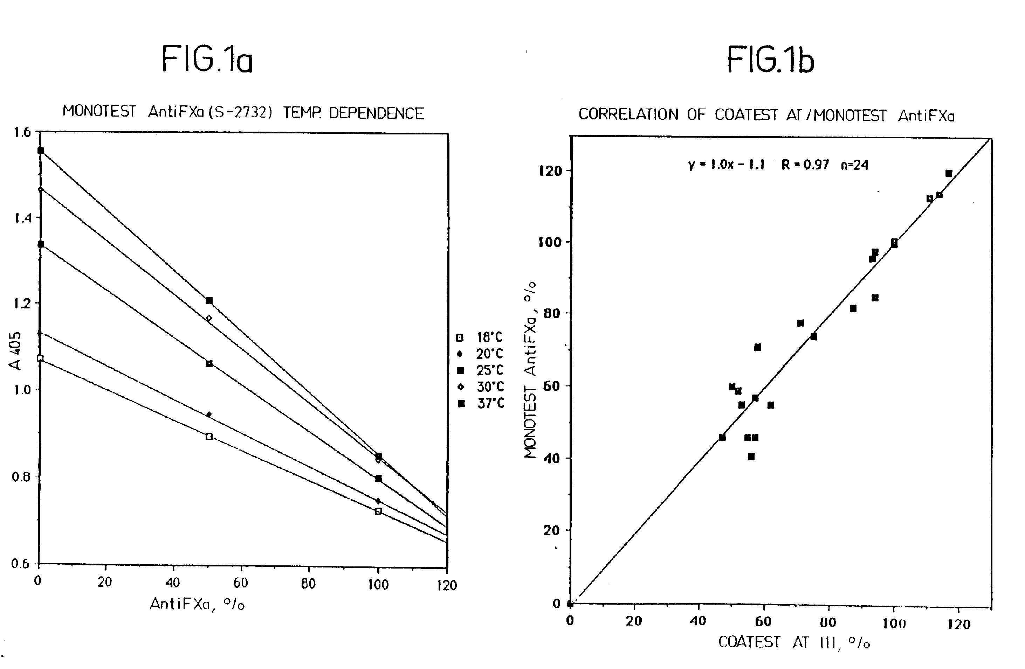

The process according to the invention is explained in greater detail by means of the following examples, which are not limiting per se, with reference to the enclosed drawings, wherein Figs. 1-9 show standard curves for a number of different components active in proteolysis and established at a wavelength of 405 nm by the aid of suitable freeze-dried reagent combinations according to the invention.

Example 1

In this example a process for the preparation of a freeze-dried reagent combination for determination of a protease inhibitor, antifactor Xa and its use are described.

a) Acetate buffer, 1000 ml, is prepared by diluting 500 ml of a mixture of 0.2 M acetic acid and 0.2 M sodium acetate in water to 1000 ml by addition of distilled water. The desired pH-value of 4.2 is obtained with 368 ml 0.2 M acetic acid and 132 ml 0.2 M sodium acetate.

b) in order to obtain an improved stability and increased solubility of the reagent combination albumin and mannitol are added to the buffer solution a) in the amounts indicated below.

c) Preparation for freeze drying:

The substrate S-2732, 650 mg/l (0.8 mmol/l) and factor Xa, 1000 nkat (1000 nkat/l) are added to the buffer solution a), to which BSA, 5 g/l (0.5 %) and mannitol, 10 g/l (1 %) have been added.

d) Preparation of a freeze-dried reagent combination intended for determination of antifactor Xa:

Each 200 µl of the solution c) is transferred to the respective microcuvette of plastic (Kartell Art. No 1938). The cuvettes are placed in a freeze-drier at -42°C for one hour and dried for 15 h at +22°C and a pressure of 0.08-0.15 mbar.

e) Use of cuvettes d) for determination of antifactor Xa:

300 µl of plasma (diluted 1:500) in 0.05 mol/l Tris, pH 8.4, I=0.2 containing EDTA (7.5 mmol/l), heparin (3 IU/ml] and PEG (1 %) are added to the cuvette d) containing the freeze-dried reagent combination consisting of S-2732 (0.13 mg) FXa (0.2 nkat) and BSA and mannitol.

After addition the pH of the test solution is 8.2. The reaction is allowed to take place at room temperature (25°C) or at 37°C and is interrupted after 8 min through addition of 300 µl 5 % AcOH.

The absorbance of the solution at 405 nm is thereafter read in a photometer and the result is compared with samples containing antifactor Xa in known amounts, by means of which the standard curves shown i Fig. 1a have already been prepared at the manufacturer of the reagent combination. This curves show the relationship between dose and response and are the basis of factors calculated by the manufacturer and utilized by the user for calculation of the analyte in question.

The reaction was found to have a low temperature dependence which is also apparent from Fig. 1a.

The correlation to a generally used and commercially available "kit" for determination of antithrombin (Coatest® Antithrumbin, KabiVitrum AB, Sweden) is shown in Fig. 1b.

Example 2

In this example a process for the preparation of a reagent combination intended for determination of antithrombin and its use are described.

a) A freeze-dried reagent combination intended for determination of antithrombin and containing S-2366 (0.1 mg), thrombin (0.7 nkat) as well as BSA and mannitol is prepared in a cuvette in analogy with example 1.

b) Use of the reagent combination in determination of antithrombin.

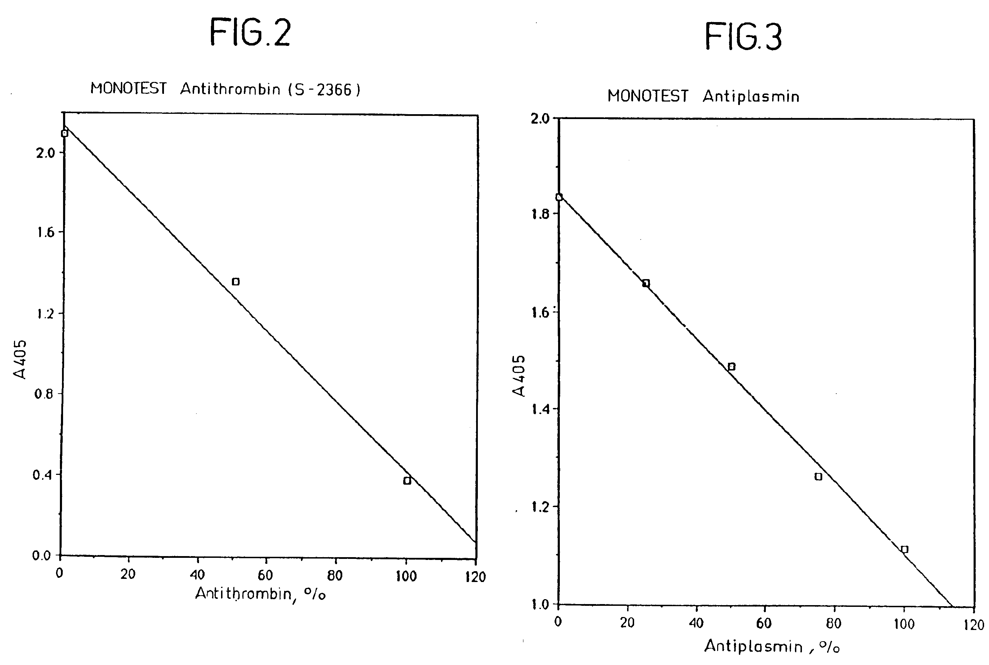

300 µl plasma (diluted 1:80) in the same buffer as indicated in example 1e) is added to the cuvette from a), the contents of which being freeeze-dried. The reaction is allowed to take place at 37°C and interrupted after 8 min through addition of 300 µl of 5 % acetic acid. Measuring is carried out in analogy with example 1e), the standard curve shown in Fig. 2 being obtained.

Example 3

In this example a process for the preparation of a reagent combination intended for determination of antiplasmin and its use are described.

a) A cuvette containing a freeze-dried reagent combination intended for determination of antiplasmin and containing S-2251 (0.3 mg), plasmin (0.3 nkat) and BSA and mannitol is prepared in analogy with example 1.

b) Use of the reagent combination in determination of antiplasmin.

300 μl plasma (diluted 1:30) in 0.05 mol/l Tris, pH = 8.3 comprising 0.15 mol/l methylamine is added to the cuvette from a), the contents of which being freeze-dried. The reaction takes place at 37°C and is interrupted after 8 min by addition of 300 μl of 5 % AcOH. Measurement is carried out in analogy with exemple 1e). The standard curve shown in Fig. 3 is obtained.

The above examples show reagent combinations intended for determination of protease inhibitors. The two immediately following examples illustrate reagent combinations intended for determination of cofactors.

Example 4

A process for the preparation of a reagent combination for determination of heparin and its use are described in this example.

a) A cuvette compring a reagent combination for determination of heparin and containing S-2732 (0.2 mg), FXa (0.5 nkat) and BSA and mannitol is prepared in analogy with example 1.

b) Use of the reagent combination in determination of heparin.

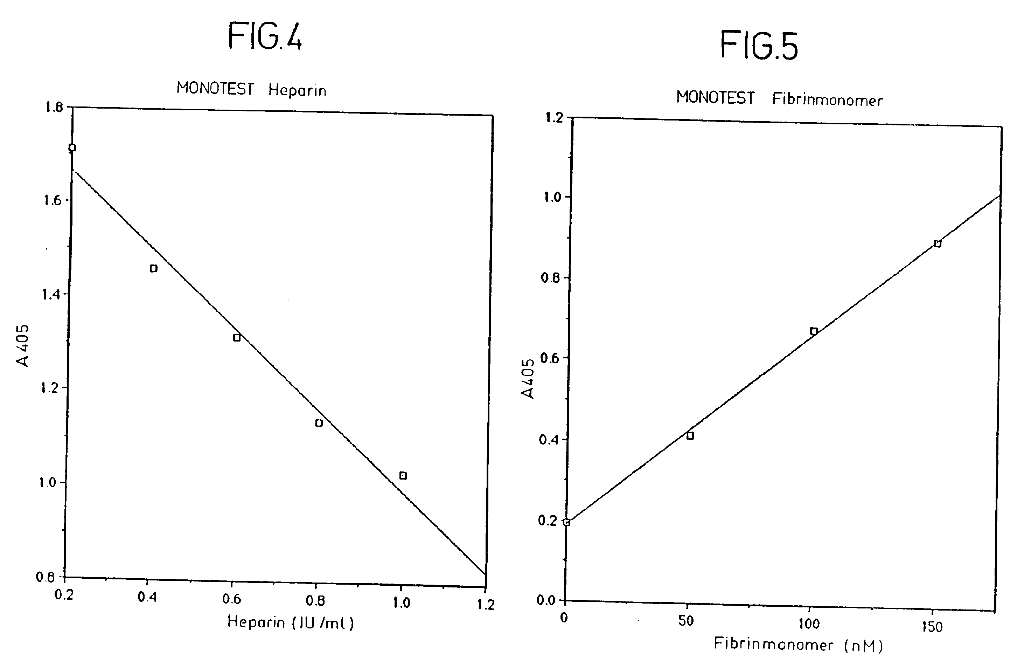

300 μl plasma (diluted 1:15) in 0.05 mol/l Tris, pH = 8.4, I = 0.2 containing EDTA (7.5 mmol/l) and PEG (1 %) is added to the cuvette from a), the contents of which being freeze-dried. The reaction is carried out at room temperature and interrupted after 6 min through addition of 300 μl of 5 % acetic acid. It is measured in analogy with example 1e). The standard curve shown in Fig. 4 is obtained.

Example 5

In this example a process for the preparation of a reagent combination for determination of fibrin monomer and its use are described.

a) S-2390 (12 mg) and mannitol (19 mg) are dissolved in 7.0 ml 0.03 mol/l sodium acetat buffer, pH = 4.9 containing 0.01 % Tween$^R$ 80 and mixed with 2.5 mg human Glu-plasminogen dissolved in 0.8 ml sterile water. 100 mg BSA dissolved in 0.5 ml water and 3.6 μg t-PA dissolved in 0,8 ml sodium acetate buffer are added to the resulting solution after which additional buffer is added so that a total volume of 20 ml is achieved. Portions of 200 μl of the final solution are distributed on microcuvettes and freeze-dried according to example 1d).

b) Use of the reagent combination in determination of fibrin monomer.

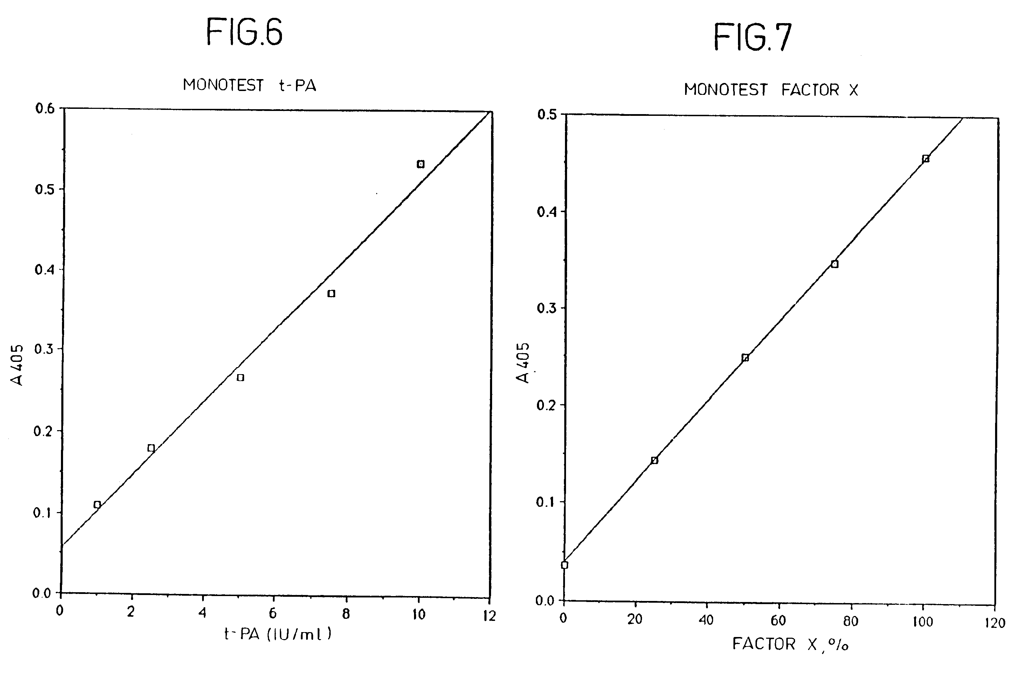

300 μl plasma (diluted 1:41) in 0.063 mol/l Tris, pH = 8.5 containing 0.01 % Tween® 80 is added to the cuvette from a), the contents of which being freeze-dried and consisting of S-2390 (0.12 mg), plasminogen (25 μg) and t-PA (0.036 μg) as well as mannitol, Tween® 80 and BSA. The reaction takes place at room temperature and is interrupted after 20 min by addition of 300 μl of 20 % acetic acid. It is measured in analogy with example 1e). The standard curve shown in Fig. 5 is obtained.

An embodiment intended for determination of an activator for an enzyme reaction is illustrated in the following example.

Example 6

A process for the preparation of a reagent combination for determination of t-PA and its use are described in this example.

a) S-2251 (9 mg) dissolved in 13.5 ml distilled water is mixed with 0.75 ml of an aqueous solution containing plasminogen (18.75 CU) and mannitol (15 mg). The resulting solution is cooled and mixed with 0.75 ml of an aqueous solution containing CNBr-digested fibrin(ogen) (3.75 mg) and mannitol (15 mg). Portions of 200 μl of the final solution are distributed on microcuvettes and freeze-dried according to example 1d).

b) Use of the reagent combination in determination of t-PA.

300 μl of pretreated plasma (diluted 1:125) in 0.05 mol/l Tris, pH = 8.3 containing 0.01 % Tween® 80 is added to the cuvette from a), the contents of which being freeze-dried and consisting of S-2251 (120 μg), plasminogen (0.25 CU) and CNBr-digested fibrin(ogen) (50 μg) as well as mannitol. The reaction is allowed to take place at 37°C and interrupted after 2 h and 45 min through addition of 300 µl 20 % AcOH. It is measured in analogy with example 1e) and the standard curve shown in Fig. 6 is obtained. Embodiments intended for determination of proenzymes are illustrated in the following example.

Example 7

A process for the preparation of a reagent combination for determination of factor X and its use are described in this example.

a) S-2337 (24 mg) and mannitol (120 mg) dissolved in 6.5 ml of distilled water are mixed with 3.5 ml of an aqueous solution containing Russel's Viper Venom (RVV, Miami Serpentarium Labs, USA) (0.9 mg) and NaCl (60 mg). A solution of $CaCl_2$ (0.1 mol/l) is added to the resulting solution so that a total volume of 20 ml is obtained. Portions of 200 µl of the final solution are distributed on microcuvettes and freeze-dried according to example 1d).

b) Use of the reagent combination in determination of factor X.

600 µl of plasma (diluted 1:60) in 0.05 mol/l Tris, pH = 7.8 containing Polybrene® (20 mg/l, Aldrich, USA) are added to the cuvette from a), the contents of which being freeze-dried and consisting of S-2337 (0.24 mg) and RVV (9 µg) as well as mannitol, $CaCl_2$ and NaCl. The reaction takes place at 37°C and is interrupted after 3 min. through addition of 200 µl of 20 % AcOH. It is measured in analogy with example 1e) and the standard curve shown in Fig. 7 is obtained.

Example 8

In this example a process for the preparation of a reagent combination for determination of plasminogen and its use are described.

a) S-2251 (30 mg) is dissolved in 16 ml of distilled water and mixed with 4 ml of a solution containing streptokinase (80 000 IU) and 12 mg of albumin (20 %). Portions of 200 µl of the resulting final solution are distributed on microcuvettes and freeze-dried according to example 1d).

b) Use of the reagent combination in determination of plasminogen.

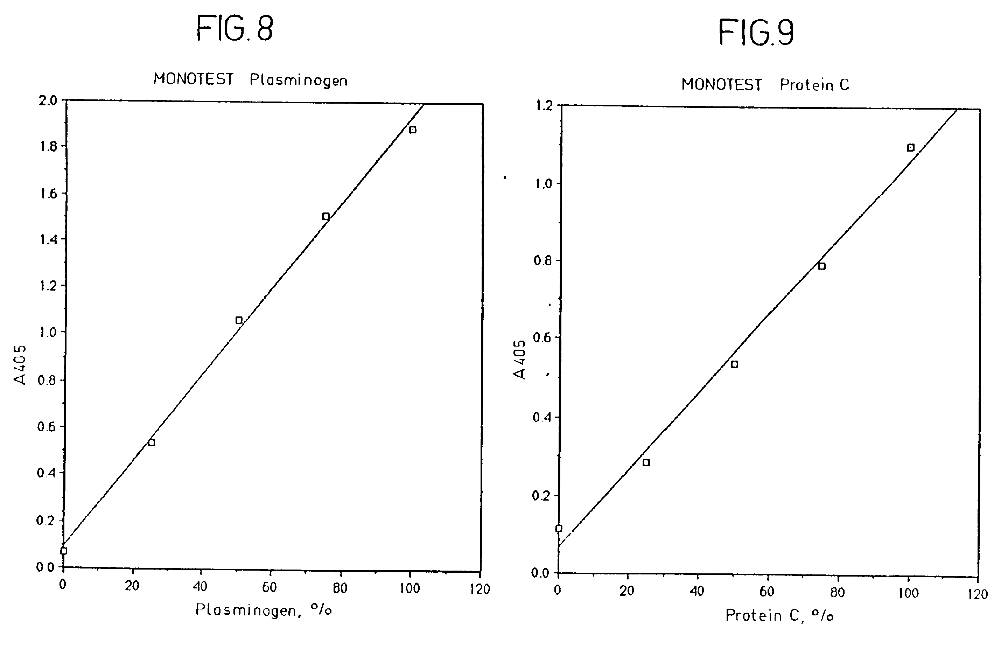

300 µl of plasma (diluted 1:20) in 0.05 mol/l Tris, pH = 7.4 and I = 0.05 are added to the cuvette from a), the contents of which being freeze-dried and consisting of S-2251 (0.3 mg) and Streptokinase (800 IU) as well as albumin. The reaction is allowed to take place at 37°C and is interrupted after 3 min by addition of 300 µl 5 % AcOH. It is measured in analogy with example 1e) and the standard curve shown in Fig. 8 is obtained.

Example 9

In this example a process for the preparation of a reagent combination for determination of protein C and its use are described.

a) S-2366 (12 mg) is dissolved in 19.55 ml of distilled water and 0.45 ml of Protac$^R$ C-solution (10 U/ml), Pentapharm, Switzerland) is added. Portions of 200 µl of the resulting solution are distributed on microcuvettes and freeze-dried according to example 1d).

b) Use of the reagent combination in determination of protein C.

300 µl plasma (diluted 1:11) in 0.025 mol/l Tris, pH = 8.4 containing 0.1 % PEG are added to the cuvette from a), the contents of which being freeze-dried and consisting of S-2366 (0.12 mg) and Protac®C (0.045 U). The reaction is allowed to take place at 37°C and is interrupted after 7 min through addition of 300 µl 20 % AcOH. It is measured in analogy with example 1e) and the standard curve shown in Fig. 9 is obtained.

In these examples abbreviations have been used having the following meanings

| | |
|---|---|
| BSA | bovine serum albumin |
| Tris | tris(hydroxymethyl)-aminomethane |
| EDTA | ethylenediamine tetraacetic acid |
| PEG | polyethylene glycol |
| AcOH | acetic acid |
| Tween®80 | polyoxyethylene sorbitan monooleate (Atlas Chemical Industries, USA) |
| Glu | glutamic acid |
| t-PA | plasminogen activator |
| S-2251 | H-D-Val-Leu-Lys-pNA·2HCl |
| S-2337 | Nα-benzoyl-Ile-Glu(γ-piperidide)-Gly-Arg-pNA·HCl |
| S-2366 | <Glu-Pro-Arg-pNA·HCl |

| S-2390 | H-D-Val-Phe-Lys-pNA·2HCl |
| S2732 | Nα-succinoyl-Ile-Glu(γ-piperidide)-Gly-Arg-pNA·HCl |
| -pNA | p-nitroanilide |
| $A_{405}$ | absorbance at 405 nm |
| nkat | nanokatal (1 katal = the amount of enzyme activity that splits 1 mol substrate per sec. under specified conditions) |
| 1 U (Heparsin) | unit related to international standard |
| 1U (Streptokinase) | unit related to international standard |
| CU (plasminogen) | casein unit |
| U (Protac®) | 1 U = the amount of Protac® activating protein C included in 1 ml of normal human citrate plasma. |

The standard curves shown on the drawings have been obtained, preferably at the manufacturer of the reagent combinations, by measurement of $A_{405}$ for samples having varying known amounts of the component to be determined by means of the respective standard curve in analogy with the measurements carried out in the corresponding illustrative example.

## Claims

1. Freeze-dried reagent combination enclosed in a container and intended for determination of a component active in proteolysis directly or indirectly by cleaving of a substrate included in the reagent combination and capable of being cleaved by a protease to produce a detectable response, **characterized** in that the reagent combination comprises in a substantially unreacted form all reagents required for the determination, said reagents comprising a chromogenic substrate and a component having enzymatic activity towards said substrate and optionally one or more additives known per se; and is prepared by freeze-drying in a way known per se a solution containing all the constituents included in the freeze-dried reagent combination in a substantially unreacted form, said freeze-dried combination having substantially its original activity at reconstitution as in a buffer solution.

2. Reagent combination as claimed in claim 1, **characterized** in that an amount of the reagent combination intended for one single determination is enclosed in a container having an undivided interior volume, which container preferably can be used directly in a measuring apparatus intended for the response detection.

3. Reagent combination as claimed in claim 1 or 2 intended for determination of a component active in proteolysis and consisting of a) an inhibitor, b) a cofactor, c) an activator or d) a proenzyme, **characterized** in that in addition to the substrate, capable of being cleaved by proteases and preferably being a chromogenic substrate, and possible additives it contains a) a protease, b) a protease and a reactant capable of reacting with the protease under the influence of the cofactor to be determined, c) a proenzyme or an enzyme compound capable of being activated or d) an activator for the proenzyme.

4. Reagent combination as claimed in claim 3, **characterized** in that it is intended for determination of 1) antifactor $X_a$, 2) heparin, 3) fibrin monomer or 4) protein C and comprises 1) factor $X_a$, Nα-succinoyl-Ile-Glu(γ-piperidide)-Gly-Arg-pNA-substrate, bovine serum albumin and mannitol; 2) factor $X_a$, Nα-succinoyl-Ile-Glu(γ-piperidide)-Gly-Arg-pNA-substrate, bovine serum albumin and mannitol, 3) plasminogen, H-D-Val-Phe-Lys-pNA-substrate, t-PA, mannitol, surfactant and bovine serum albumin or 4) protein C-activator and <Glu-Pro-Arg-pNA-substrate.

5. Process for the preparation of a freeze-dried reagent combination according to claim 1, which reagent combination is intended for determination of components active in proteolysis directly or indirectly through cleaving of a substrate included in the reagent combination and capable of being cleaved by a protease to produce a detectable response, **characterized** in that a solution containing all the reagents required for the determination, said reagents comprising a chromogenic substrate and a component having enzymatic activity towards said substrate and optionally one or more additives known per se, is produced in a container in a solvent such as water under conditions controlled in such a way that the reagents remain substantially nonreactive in the resulting solution and in a following freeze-drying of the solution; and that the solution is freeze-dried in the container in a way known per se, the freeze-dried reagent combination being obtained wherein the reagents are included in a substantially unreacted form and wherein the reagents have substantially original activity after reconstitution of the reagent combination such as in a buffer solution.

6. Process as claimed in claim 5, **characterized** in that the container contains such a quantity of solution in the freeze drying as is suitable for one single determination, said container having an undivided interior volume.

7. Process as claimed in claim 5 or 6, **characterized** in that the conditions are controlled, preferably by means of a buffer, to a pH-value of 3-8.5 when a reagent combination intended for determination of cofactors or activators is prepared, and a pH-value of 3-5 when a reagent combination intended for determination of inhibitors or proenzymes is prepared.

8. Process as claimed in claim 5, 6 or 7, **characterized** in that the freeze-drying is carried out at 0.08-0.15 mbar through freezing at a temperature of -45° to -40°C, preferably -42°C for a time of 1-5 h, preferably 1 h, and a subsequent drying for a time of 12-20 h, preferably 15 h, at a temperature of 12-24°C, preferably 22°C.

9. Process as claimed in any of the preceding claims 5-8, **characterized** in that the solution contains additives, such as inactive protein, for example albumin; sugar, for example mannitol; and/or surfactant, for example polyethylene glycol.

10. Process as claimed in any of the preceding claims 5-9, **characterized** in that a reagent combination is prepared intended for determination of a component active in proteolysis and consisting of a) an inhibitor, b) a cofactor, c) an activator or d) a proenzyme and that the solution of the reagent combination, which is freeze-dried, contains in addition to the substrate capable of being cleaved by protease, and possible additives a) a protease, b) a protease and a reactant which can react with the protease under the influence of the cofactor to be determined, c) a proenzyme or an enzyme compound capable of being activated or d) an activator for the proenzyme.

11. Use of a freeze-dried reagent combination as claimed in claim 1 for quantitative, semi-quantitative or qualitative determination of coagulation factors and fibrinolysis factors in a sample, especially a biological one, such as whole blood, blood plasma, blood serum, cerebrospinal liquid, lung liquid or urine in a one-step process through addition of the sample to the freeze-dried reagent combination enclosed in a container, preferably a cuvette, and reading of the resulting response in a way known per se.

## Patentansprüche

1. Gefriergetrocknete Reagentienkombination, die in einem Behälter eingeschlossen und zur Bestimmung einer bei der Proteolyse wirksamen Komponente vorgesehen ist, und zwar direkt oder indirekt durch Spaltung eines in der Reagentienkombination enthaltenen, durch eine Protease unter Bewirkung einer meßbaren Antwort spaltbaren Substrats, dadurch gekennzeichnet, daß die Reagentienkombination in im wesentlichen nicht-reagierter Form sämtliche für die Bestimmung erforderlichen Reagentien enthält, wobei die Reagentien ein chromogenes Substrat und eine Komponente mit enzymatischer Aktivität gegenüber dem Substrat sowie gegebenenfalls ein an sich bekanntes Additiv oder mehrere umfassen, und daß sie durch eine an sich bekannte Gefriertrocknung einer sämtliche in der gefriergetrockneten Reagentienkombination in im wesentlichen nicht-reagierter Form enthaltenden Lösung hergestellt ist, wobei die gefriergetrocknete Kombination bei ihrer Rekonstitution, z.B. in einer Pufferlösung, im wesentlichen ihre ursprüngliche Aktivität aufweist.

2. Reagentienkombination nach Anspruch 1, dadurch gekennzeichnet, daß in einem Behälter mit einem nicht unterteilten Innenraum eine Menge der für eine Einzelbestimmung vorgesehenen Reagentienkombination enthalten ist, wobei der Behälter bevorzugt direkt in einer für die Bestimmung der Antwort vorgesehen Meßvorrichtung einsetzbar ist.

3. Reagentienkombination nach Anspruch 1 oder 2, die zur Bestimmung einer in der Proteolyse wirksamen Komponente vorgesehen ist und die aus a) einem Inhibitor, b) einem Cofaktor, c) einem Aktivator oder d) einem Proenzym besteht, dadurch gekennzeichnet, daß sie zusätzlich zu dem Substrat, das durch Proteasen spaltbar und bevorzugt ein chromogenes Substrat ist, und gegebenenfalls anderen Additiven a) eine Protease, b) eine Protease und ein mit der Protease unter dem Einfluß des zu bestimmenden Cofaktors reaktionsfähiges Reagens, c) ein Proenzym oder eine aktivierbare Enzymverbindung oder d) einen Aktivator für das Proenzym enthält.

4. Reagentienkombination nach Anspruch 3, dadurch gekennzeichnet, daß sie zur Bestimmung von 1) Antifaktor $X_a$, 2) Heparin-, 3) Fibrinmonomer oder 4) Protein C vorgesehen ist und
    1) Faktor $X_a$, N-alpha-Succinoyl-Ile-Glu(gamma-piperidid)-Gly-Arg-pNA-Substrat, Rinder-Serumalbumin und Mannitol;
    2) Faktor $X_a$, N-alpha-Succinoyl-Ile-Glu(gamma-piperidid)-Gly-Arg-pNA-Substrat, Rinder-Serumalbumin und Mannitol;
    3) Plasminogen, H-D-Val-Phe-Lys-pNA-Substrat, t-PA, Mannitol, oberflächenaktives Mittel und Rinder-Serumalbumin oder
    4) Protein C-Aktivator und Glu-Pro-Arg-pNA-Substrat
enthält.

5. Verfahren zur Herstellung einer gefriergetrockneten Reagentienkombination nach Anspruch 1, die zur Bestimmung von bei der Proteolyse wirksamen Komponenten vorgesehen ist, und zwar direkt oder indirekt durch Spaltung eines in der Reagentienkombination enthaltenen, durch eine Protease unter Bewirkung einer meßbaren Antwort spaltbaren Substrats, dadurch gekennzeichnet, daß eine sämtliche für die Bestimmung erforderlichen Reagentien enthaltende Lösung, wobei die Reagentien ein chromogenes Substrat und eine Komponente mit enzymatischer Aktivität gegenüber dem Substrat sowie gegebenenfalls ein an sich bekanntes Additiv oder mehrere umfassen, in einem Behälter in einem Lösemittel, z.B. Wasser, unter so kontrollierten Bedingungen hergestellt wird, daß die Reagentien in der sich ergebenden Lösung und bei einem sich anschließenden Gefriertrocknen der Lösung im wesentlichen nicht-reaktiv bleiben, und daß die Lösung in dem Behälter in an sich bekannter Weise gefriergetrocknet wird, wobei die gefriergetrocknete Reagentienkombination erhalten wird, und wobei die Reagentien in einer im wesentlichen nicht-reagierten Form enthalten sind und im wesentlichen nach der Rekonstitution der Reagentienkombination, z.B. in einer Pufferlösung, im wesentlichen die ursprüngliche Aktivität aufweisen.

6. Verfahren nach Anspruch 5, dadurch gekennzeichnet, daß der Behälter bei dem Gefriertrocknen die Lösung in einer Menge enthält, die für eine Einzelbestimmung geeignet ist, wobei der Behälter einen nicht unterteilten Innenraum aufweist.

7. Verfahren nach Anspruch 5 oder 6, dadurch gekennzeichnet, daß bei der Herstellung einer für die Bestimmung von Cofaktoren oder Aktivatoren vorgesehenen Reagentienkombination die Bedingungen, vorzugsweise mittels eines Puffers, auf einen pH-Wert von 3 bis 8,5, und bei der Herstellung einer für die Bestimmung von Inhibitoren oder Proenzymen vorgesehenen Reagentienkombination auf einen pH-Wert von 3 bis 5 eingestellt werden.

8. Verfahren nach Anspruch 5, 6 oder 7, dadurch gekennzeichnet, daß die Gefriertrocknung bei 0,08 bis 0,15 mbar unter Gefrieren bei einer Temperatur von -45° bis -40°C, vorzugsweise -42°C, für eine Zeitspanne von 1 bis 5 h, vorzugsweise 1 h, und einem anschließenden Trocknen für eine Zeitspanne von 12 bis 20 h, vorzugsweise 15 h bei einer Temperatur von 12 bis 24°C, vorzugsweise 22°C, durchgeführt wird.

9. Verfahren nach einem jeden der obigen Ansprüche 5 bis 8, dadurch gekennzeichnet, daß die Lösung Additive, wie inaktives Protein, z.B. Albumin; Zucker bzw. Kohlenhydrate, z.B. Mannitol; und/oder oberflächenaktive mittel, z.B. Polyethylenglykol, enthält.

10. Verfahren nach einem jeden der obigen Ansprüche 5 bis 9, dadurch gekennzeichnet, daß man eine bei der Bestimmung einer bei der Proteolyse wirksamen Reagentienkombination herstellt, die aus a) einem Inhibitor, b) einem Cofaktor, c) einem Aktivator oder d) einem Proenzym besteht, und daß die Lösung der Reagentienkombination, die gefriergetrocknet wird, zusätzlich zu dem durch Protease spaltbaren Substrat und gegebenenfalls Additiven a) eine Protease, b) eine Protease und einen mit der Protease unter dem Einfluß des zu bestimmenden Cofaktors zur Reaktion befähigten Reaktanten, c) ein Proenzym oder eine aktivierbare Enzymverbindung oder d) einen Aktivator für das Proenzym enthält.

11. Verwendung einer gefriergetrockneten Reagentienkombination nach Anspruch 1 für die quantitative, semi-quantitative oder qualitative Bestimmung von Coagulationsfaktoren und Fibrinolysefaktoren in einer Probe, insbesondere einer biologischen Probe, z.B. Vollblut, Blutplasma, Blutserum, cerebrospinaler Flüssigkeit, Lungenflüssigkeit oder Urin, in einem einstufigen verfahren durch Zugabe der Probe zu der in einem Behälter, vorzugsweise einer Küvette, enthaltenen gefriergetrockneten Reagentienkombination, und durch Registrierung der sich ergebenden Reaktion bzw. Antwort in an sich bekannter Weise.

## Revendications

1. Composition de réactifs lyophilisée contenue dans un récipient et destinée à la détermination directe ou indirecte d'un constituant à activité protéolytique par dissociation d'un substrat contenu dans la composition de réactifs et susceptible d'être dissocié par une protéase pour produire une réponse décelable, caractérisée en ce que cette composition de réactifs comprend sous une forme essentiellement inaltérée tous les réactifs nécessaires à la détermination, lesdits réactifs comprenant un substrat chromogène et un constituant ayant une activité enzymatique à l'égard dudit substrat et éventuellement un ou plusieurs additifs connus en soi; et en ce qu'elle est préparée par lyophilisation d'une façon connue en soi d'une solution contenant tous les constituants compris dans la composition de réactifs lyophilisée sous une forme essentiellement inaltérée, ladite composition lyophilisée ayant pratiquement toute son activité d'origine au moment de la reconstitution par exemple dans une solution de tampon.

2. Composition de réactifs selon la revendication 1, caractérisée en ce qu'une quantité de la composition de réactifs destinée à une détermination unique est contenue dans un récipient ayant un volume intérieur non divisé, ce récipient pouvant être utilisé de préférence directement dans un appareil de mesure destiné à déceler la réponse.

3. Composition de réactifs selon la revendication 1 ou 2, destinée à la détermination d'un constituant à activité protéolytique consistant en a) un inhibiteur, b) un cofacteur, c) un activateur ou d) une pro-enzyme, caractérisée en ce que, en plus du substrat susceptible d'être dissocié par les protéases et étant de préférence un substrat chromogène, et d'additifs éventuels, elle contient a) une protéase, b) une protéase et un réactif susceptible de réagir avec la protéase sous l'influence du cofacteur à déterminer, c) une pro-enzyme ou un composé enzymatique susceptible être activé ou d) un activateur pour la pro-enzyme.

4. Composition de réactifs selon la revendication 3, caractérisée en ce qu'elle est destinée à la détermination de 1) l'antifacteur $X_a$, 2) l'héparine, 3) un monomère de la fibrine ou 4) la protéine C et comprend 1) le facteur $X_a$, le substrat N$\alpha$-succinoyl-Ile-Glu($\gamma$-pipéridide)-Gly-Arg-pNA, de la sérumalbumine bovine et du mannitol; 2) le facteur $X_a$, le substrat N$\alpha$-succinoyl-Ile-Glu($\gamma$-pipéridide)-Gly-Arg-pNA, de la sérumalbumine bovine et du mannitol; 3) du plasminogène, le substrat H-D-Val-Phe-Lys-pNA, du t-PA, du mannitol, un agent tensio-actif et de la sérumalbumine bovine, ou 4) un activateur de la protéine C et le substrat <Glu-Pro-Arg-pNA.

5. Procédé de préparation d'une composition de réactifs lyophilisée selon la revendication 1, cette composition de réactifs étant destinée à la détermination directe ou indirecte de constituants ayant une activité protéolytique par dissociation d'un substrat, compris dans la composition de réactifs, susceptible d'être dissocié par une protéase pour produire une réponse décelable, caractérisé en ce qu'on prépare dans un récipient une solution contenant tous les réactifs nécessaires à la détermination, lesdits réactifs comprenant un substrat chromogène et un constituant ayant une activité enzymatique à l'égard dudit substrat et éventuellement un ou plusieurs additifs connus en soi, dans un solvant tel que l'eau dans des conditions contrôlées de façon à ce que les réactifs restent essentiellement non réactifs dans la solution obtenue et au cours de la lyophilisation ultérieure de cette solution; et en ce qu'on lyophilise la solution dans le récipient d'une façon connue en soi, la composition de réactifs lyophilisée obtenue contenant les réactifs sous une forme essentiellement inaltérée et ayant pratiquement leur activité d'origine après la reconstitution de la composition de réactifs dans une solution de tampon par exemple.

6. Procédé selon la revendication 5, caractérisé en ce que le récipient contient au cours de la lyophilisation une quantité de solution telle qu'elle convient pour une détermination unique, ledit récipient ayant un volume intérieur non divisé.

7. Procédé selon la revendication 5 ou 6, caractérisé en ce que les conditions sont contrôlées, de préférence au moyen d'un tampon, à une valeur de pH de 3-8,5 quand on prépare une composition de réactifs destinée à la détermination de cofacteurs ou d'activateurs, et à une valeur de pH de 3-5 quand on prépare une composition de réactifs destinée à la détermination d'inhibiteurs ou de pro-enzymes.

8. Procédé selon la revendication 5,6 ou 7, caractérisé en ce qu'on effectue la lyophilisation à 0,08-0,15 mbars par congélation à une température de -45° à -40°C, de préférence de -42°C pendant une période de 1-5 h, de préférence d'1 h, puis séchage pendant une période de 12-20 h, de préférence de 15 h, à une température de 12-24°C, de préférence de 22°C.

9. Procédé selon l'une quelconque des revendications 5-8 précédentes, caractérisé en ce que la solution contient des additifs tels qu'une protéine inactive, par exemple une albumine; un sucre, par exemple du mannitol; et/ou un agent tensio-actif, par exemple du polyéthylèneglycol.

10. Procédé selon l'une quelconque des revendications 5-9 précédentes, caractérisé en ce qu'on prépare une composition de réactifs destinée à la détermination d'un constituant à activité protéolytique consistant en a) un inhibiteur, b) un cofacteur, c) un activateur ou d) une pro-enzyme, et en ce que la solution de la composition de réactifs qu'on lyophilise contient, en plus du substrat susceptible d'être dissocié par les protéases et d'additifs éventuels, a) une protéase, b) une protéase et un réactif susceptible de réagir avec la protéase sous l'influence du cofacteur à déterminer, c) une pro-enzyme ou un composé enzymatique susceptible être activé ou d) un activateur pour la pro-enzyme.

11. Utilisation d'une composition de réactifs lyophilisée selon la revendication 1 pour la détermination quantitative, semi-quantitative ou qualitative de facteurs de coagulation et de facteurs de fibrinolyse dans un échantillon, notamment biologique, tel que du sang total, du plasma sanguin, du sérum sanguin, du liquide cérébro-spinal, du liquide pulmonaire ou de l'urine en un procédé en une étape par addition de l'échantillon à la composition de réactifs lyophilisée contenue dans un récipient, de préférence dans une cuvette, et lecture de la réponse obtenue par un moyen connu en soi.

# FIG.1a

## MONOTEST AntiFXa (S-2732) TEMP. DEPENDENCE

Legend:
- □ 18°C
- ◆ 20°C
- ■ 25°C
- ◇ 30°C
- ■ 37°C

X-axis: AntiFXa, %
Y-axis: A 405

# FIG.1b

## CORRELATION OF COATEST AT/MONOTEST AntiFXa

$y = 1.0x - 1.1 \quad R = 0.97 \quad n=24$

X-axis: COATEST AT III, %
Y-axis: MONOTEST AntiFXa, %

EP 0 318 571 B1

# FIG.2

## MONOTEST Antithrombin (S-2366)

# FIG.3

## MONOTEST Antiplasmin

EP 0 318 571 B1

# FIG.4

MONOTEST Heparin

# FIG.5

MONOTEST Fibrinmonomer

EP 0 318 571 B1

# FIG.6

MONOTEST t-PA

# FIG.7

MONOTEST FACTOR X

EP 0 318 571 B1

FIG. 8

MONOTEST  Plasminogen

FIG.9

MONOTEST  Protein C

EP 0 318 571 B1